(19) Europäisches Patentamt

European Patent Office

Office européen des brevets

(11) Veröffentlichungsnummer: **0 263 360 B1**

(12) **EUROPÄISCHE PATENTSCHRIFT**

(45) Veröffentlichungstag der Patentschrift: **13.05.92**

(51) Int. Cl.⁵: **A61B 17/12**, A61F 5/00

(21) Anmeldenummer: **87113902.8**

(22) Anmeldetag: **23.09.87**

(54) **Clip zum Verschliessen eines Eileiters, Samenleiters oder eines Blutgefässes.**

(30) Priorität: **06.10.86 DE 3633974**

(43) Veröffentlichungstag der Anmeldung:
**13.04.88 Patentblatt 88/15**

(45) Bekanntmachung des Hinweises auf die
Patenterteilung:
**13.05.92 Patentblatt 92/20**

(84) Benannte Vertragsstaaten:
**CH DE FR GB LI NL SE**

(56) Entgegenhaltungen:
**DE-A- 3 334 801**
**DE-C- 3 445 874**
**US-A- 3 726 279**

**SOVIET INVENTION ILLUSTRATED, 30. Januar
1985, Seite 31, Zusammenfassung Nr.
311354, Derwent Publications Ltd., Section
Mechanical, Woche 8450 & SU-A - 1088-713
(DON MEDICAL INST) 30.04.1984**

(73) Patentinhaber: **Bleier, Waldemar Dr.Med.
Rodener Schanze 6
W-6630 Saarlouis(DE)**

(72) Erfinder: **Bleier, Waldemar Dr.Med.
Rodener Schanze 6
W-6630 Saarlouis(DE)**

(74) Vertreter: **Moser, Herbert, Dr.-Ing. et al
Nowackanlage 15
W-7500 Karlsruhe 1(DE)**

## Beschreibung

Die Erfindung betrifft einen Doppelringclip zum Verschließen eines Eileiters, Samenleiters oder eines Blutgefäßes mit zwei durch eine stegförmige Verbindung zu einer Einheit verbundenen in gleichem Abstand zueinander liegenden ringförmigen Klemmzungen.

Clips zum Verschließen von Ei-, Samenleitern oder Blutgefäßen sind zur Verwendung in der Human- und Tiermedizin für verschiedene Anwendungsfälle bekannt. Sie dienen beispielsweise als mechanische Antikonzeptionsmittel, wobei durch Abklemmen des Samenlieters das Hindurchtreten von Keimzellen verhindert wird. In gleicher weise werden derartige Clips zur Unterbrechung des Eileiters und zur andauernden oder zeitweisen Abklemmung von Blutgefäßen benutzt.

Bekannt sind z .B. aus der US-PS 39 26 195 Clips mit im wesentlichen gegeneinander bewegbaren Klemmbacken, bei denen die Blockierung des Leiters durch Quetschung erfolgt.

Günstiger erscheint ein Verschluß des Leiterlumens durch Zuschnürung wie bei einer Ligatur. Ein solcher Verschluß wird im allgemeinen als gefäßschonender angesehen. Derartige einsträngige Ringclips werden u.a. in der DE-AS 1 800 719 und in der US-PS 3 538 917 beschrieben.

Bei den durch Quetschung oder Zuschnürung verschließenden Clips besteht die Möglichkeit, daß das abgeschnürte Gewebe durch totale Druckatrophie untergeht. Dies soll durch eine Weiterbildung des Clips nach der DE-PS 34 45 874 C1 vermieden werden. Bei dieser Ausbildung wird der abzuklemmende Leiter nicht durchgehend gequetscht und damit von seinem Kontakt mit den seiner Ernährung dienenden Gefäßen abgesperrt, sondern es wird streckenweise die Gewebsernährung dadurch aufrecht erhalten, daß zwischen zwei Druckzonen beidseitig eine spaltförmige Ernährungszone offenbleibt. Eine solche Ausbildung kann jedoch die gestellten Anforderungen hinsichtlich ausreichender Ernährung des Zwischenabschnittes und gegebenenfalls eine Mikrozirkulation in den abgequetschten Bereichen zur Vermeidung einer totalen Druckatrophie nicht vollständig erfüllen.

Ein Doppelringclip der eingangs genannten Art ist in der US-PS 3 726 279 vorbeschrieben. Dabei handelt es sich um ein Formstück aus elastischem Material, wie Nylon oder Polypropylen. Ein solcher Doppelringclip erfordert eine Anpassung an den Querschnitt des abzuklemmenden Leiters und ergibt lediglich eine doppelte Klemm- bzw. Quetschwirkung, jedoch keinen einer Einschnürung (Ligatur) gleichwertigen Verschluß des Leiterlumens.

Die Erfindung geht von der Aufgabenstellung aus, einen Doppelringclip der eingangs beschriebenen Art so auszubilden, daß ein einer Ligatur gleichwertiger Verschluß des Leiterlumens und eine Anpassung an unterschiedliche Leiter ermöglicht wird.

Die Lösung dieser Aufgabenstellung erfolgt dadurch, daß die stegförmige Verbindung als gemeinsame Schließhülse ausgebildet ist an der zwei Einschnür-Zungen vorgesehen sind, welche unter veränderbarem Umfang in der gemeinsamen Schließhülse festspannbar sind. Ein solcher Doppelringclip ermöglicht durch die optimale Anpassung seiner getrennt nebeneinanderliegenden Einschnür-Zungen an das rohrförmige organische Gebilde einen günstigen einer Ligatur gleichwertigen Verschluß des Leiterlumens. Wegen der relativ großen, bis auf die stegförmige Verbindung der beiden ringförmigen Einschnür-Zungen, offenen Ernährungszone wird dabei ein nahezu allseitiger Kontakt zu den Lymph-Blut-Kapillaren und zu dem vitalisierenden Bauchhöhlen-Transsudat hergestellt. Außerdem läßt sich ein derartiger Verschluß durch die geringe Traumatisierung des Leiters reversibel ausbilden.

Die Einschnür-Zungen können zweckmäßig durch in verschiedener Weise ausbildbare Rastteile fixierbar sein. Eine vorteilhafte Ausführungsform kann vorsehen, daß die Rastteile eine Sägeverzahnung aufweisen. Diese Sägeverzahnung, die ein- oder mehrfach ausgebildet sein kann, greift in eine Schließhülse ein und ermöglicht eine der Konsistenz der zu blockierenden Leiter angepaßte Einschnürspannrung.

Durch die Zweisträngigkeit im Abstand von etwa einer Strangbreite wird eine hohe Stabilität und Ortsfestigkeit auf dem zu blockierenden Leiter erreicht. Der geringgradige Austritt der Einschnür-Zungen aus der Schließhülse nach dem Schließvorgang bedeutet keine Beeinträchtigung bei der Anwendung.

Die Platzierung des Doppelringclips erfolgt mit Hilfe eines entsprechend angepaßten Setzgerätes.

An der Oberfläche des Doppelringclips können einseitig offene Mikromulden angeordnet sein.

Der zweisträngige Ringclip kann aus einem biologisch abbaubaren Material, beispielsweise aus dem in der Chirurgie bewährten Polylactin 910 bestehen. Dadurch läßt sich erreichen, daß der Ringclip, nachdem ein dauerhafter Verschluß den Hohlorgans - in diesem Falle unter völliger Denaturierung des Gewebes durch entsprechend hohen Kompressionsdruck - erreicht ist, durch Resorption des Clipmaterials selbsttätig eliminiert wird.

Eine günstige Ausführungsform des Ringclips kann so aufgebaut sein, daß die beiden Einschnür-Zungen 1,2 mm Breite und einen gleichbleibenden Abstand von 1,2 mm aufweisen, wobei der Doppelringclip aus Kunststoff besteht.

Die Erfindung soll nachfolgend unter Bezug auf die Zeichnung näher erläutert werden; es zeigen:

Fig. 1     eine Seitenansicht eines Doppelringclips in der Ausgangslage,

Fig. 2     eine Vorderansicht des Ringclips nach Fig. 1,

Fig. 3     eine Vorderansicht des zum Aufsetzen aufgeweiteten Ringclips,

Fig. 4     einen Schnitt durch die Schließhülse längs der Linie 4-4 in Fig. 3 bei einem Ringclip nach Fig. 1,

Fig. 5     einen Querschnitt durch den zungenteil längs der Linie 5-5 in Fig. 3,

Fig. 6     eine Seitenansicht des Ringclips nach Fig. 1 in Blockierungslage,

Fig. 7     eine Vorderansicht längs der Linie 7-7 in Fig. 6,

Fig. 8     einen Ringclip mit alternativer Ausbildung der Rastvorrichtung in einer Fig. 3 entsprechenden Darstellung,

Fig. 9     einen Schnitt durch einen Eindrucksschließkopf längs der Linie 9-9 in Fig. 8,

Fig. 10    eine Vorderansicht eines zweisträngigen Ringclips mit Mikromulden.

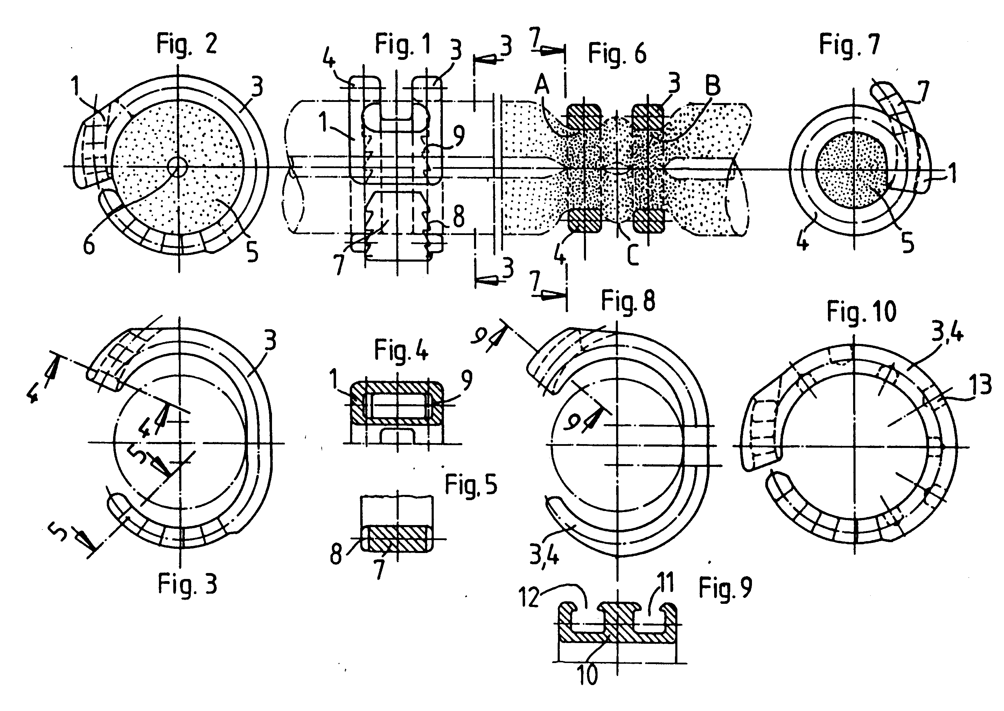

In Fig. 1 - 6 ist ein Doppelringclip dargestellt, bei dem von einer gemeinsamen Schließhülse 1 zwei in gleichem Abstand liegende Einschnür-Zungen 3,4 ausgehen, welche als ringförmige Einschnür-Zungen einen Leiter 5 umgreifen, wobei in der Darstellung der Fig. 2 zunächst ein mittleres Lumen 6 als Durchlaßöffnung freigegeben ist.

Am Ende der beiden in gleichem Abstand liegenden Einschnür-Zungen 3,4 befindet sich ein als Verbindungssteg geformter Einsteckteil 7, welcher an seinen Außenrändern eine Sägeverzahnung 8 aufweist. Dieser Sägeverzahnung 8 entspricht eine angepaßte Sägeverzahnung 9, beidseitig im freien Innenraum der Schließhülse 1.

Fig. 6 und 7 zeigen den Doppelringclip in der angezogenen Blockierungsstellung, bei der der Leiterstrang 5 durch die Einschnür-Zungen 3,4 unter Kompression und Verschluß der Durchtrittsöffnung 6 auf ca. 70 % der Masse des Querschnitts in zwei nahe beieinanderliegenden Druckzonen A und B komprimiert ist. Zwischen den beiden Druckzonen A und B befindet sich eine weitgehend offene zylinderringförmige Ernährungszone C, welche eine ausreichende Blut- und Lymphernährung der Druckzonen A und B ermöglicht. Der in Fig. 7 erkennbare geringgradige Austritt des Einsteckteils 7 aus der Schließhülse 1 beeinträchtigt die Anwendbarkeit nicht.

Eine zum Einschnüren von Ei- oder Samenleitern verwendbare Ausführungsform des zweisträngigen Ringclips besitzt in der nicht vorgespannten Ausgangslage einen äußeren Durchmesser von ca. 7 mm. Die beiden Einschnür-Zungen 3,4 sind jeweils ca. 1,2 mm stark und liegen in einem gleichmäßigen Abstand von ca. 1,2 mm. Die Dicke des gemeinsamen Einsteckteils 7 beträgt ca. 0,9 mm, während die angepaßte Ausnehmung in der Schließhülse 1 mit ca. 1 mm Höhe bei einer Gesamtdicke der Schließhülse von 1,7 mm und einer Gesamtbreite von ca. 3,6 mm festgelegt wird. Die Zahntiefe der Sägeverzahnungen 8,9 beträgt ca. 0,35 mm.

Eine alternative Ausführung nach den Figuren 8 und 9 zeigt in Fig. 8 einen Doppelringclip in einem der Darstellung nach Fig. 3 im wesentlichen entsprechenden Aufbau. Unterschiedlich ist jedoch die Ausbildung der Rastvorrichtung für die beiden freien Enden der Einschnür-Zungen 3,4. Hierbei werden die beiden Enden der Einschnür-Zungen 3,4 in eine zweinutige Eindruckschließhülse 10 (vgl. Fig. eingedrückt, wobei die beiden Nutenausnehmungen 11,12 zur sicheren Fixierung nach außen verjüngt sind. Bei einer eventuellen Notwendigkeit eines variablen Einschnürdruckes können auch bei dieser Ausführungsform die vertikalen Flanken der Nutenausnehmungen 11,12 der Eindruckschließhülse 10 und die Schließzunge mit Sägezahn-Sperrklinken versehen werden.

Das zum Einsetzen benötigte Setzgerät wird in dieser Ausführungsform der Figuren 8 und 9 in seiner Konstruktion etwas aufwendiger als das für die Ausführungsform der Figuren 1 - 7 benötigte Gerät Dies ist dadurch bedingt, daß der Schließkopf einerseits gegen die Masse des Leiters vorgespannt werden muß, damit die beiden Einschnür-Zungen, welche ein U-förmig offenes Formstück bilden, beim Schließvorgang über die Eindruckschließhülse 10 hinweggeführt werden können. Anschließend ist ein Einrasten der Einschnür-Zungen 3,4 in die beiden Nutenausnehmungen 11,12 erforderlich.

Die Darstellung nach Fig. 10 zeigt einen zweisträngigen Ringclip, in dem eine Anzahl von Mikromulden 13 eingeformt sind.

Diese Mikromulden ergeben bei allen Ausführungsformen des Ringclips eine zusätzliche Fixierung an dem um-schlossenen Leiter durch Zelleinsprossungen. Sie bewirken eine sichere Fixierung des Ringclips am Leiter, auch wenn nach einem Gewebeschwund die als Ringfurchen ausgebildeten Druckzonen völlig denaturiert und durchtrennt wären.

Die mit dem Leiter 5 in Verbindung stehenden Oberflächen des Clips bzw. der Clipteile können gegebenenfalls mit einer elastischen Beschichtung, beispielsweise in Form einer Silikon-Schicht versehen sein. Dadurch läßt sich die Kompressionskraft zusätzlich derart begrenzen, daß an den Verschlußstellen noch eine gewisse Mikrozirkulation aufrechterhalten werden kann.

## Patentansprüche

1. Doppelringclip zum Verschließen eines Eileiters, Samenleiters oder Blutgefäßesmit zwei durch eine stegförmige Verbindung zu einer Einheit verbundenen, in gleichem Abstand zueinander liegenden ringförmigen Klemmzungen, **dadurch gekennzeichnet,** daß die stegförmige Verbindung als gemeinsame Schließhülse (1;10) ausgebildet ist, an der zwei Einschnür-Zungen (3,4;) vorgesehen sind, welche unter veränderbarem Umfang in der gemeinsamen Schließhülse (1;10) festspannbar sind.

2. Doppelringclip nach Anspruch 1, **dadurch gekennzeichnet,** daß die Einschnür-Zungen (3,4) durch Rastteile (8,9; 11,12) fixierbar sind.

3. Doppelringclip nach Anspruch 2, **dadurch gekennzeichnet,** daß die Rastteile eine Sägeverzahnung (8,9) aufweisen.

4. Doppelringclip nach Anspruch 1, **dadurch gekennzeichnet,** daß die beiden Einschnür-Zungen (3,4) 1,2 mm Breite und einen gleichbleibenden Abstand von 1,2 mm aufweisen.

5. Doppelringclip nach Anspruch 1, **dadurch gekennzeichnet,** daß an der Oberfläche des Clips einseitig offene Mikromulden (13) vorgesehen sind.

6. Doppelringclip nach Anspruch 1**, dadurch gekennzeichnet,** daß der Clip aus einem biologisch abbaubaren Material besteht.

7. Doppelringclip nach Anspruch 1, **dadurch gekennzeichnet,** daß der Clip aus Kunststoff besteht.

8. Doppelringclip nach Anspruch 1, **dadurch gekennzeichnet,** daß der Clip aus Metall besteht.

9. Doppelringclip nach Anspruch 1, **dadurch gekennzeichnet,** daß die Einschnür-Zungen (3,4;) eine elastische Beschichtung aufweisen.

## Claims

1. Double ring clip for closing an oviduct, vas deferens or blood vessel having two annular clamping tongues lying an equal distance apart from one another and joined by a web-like connection to form one unit, characterized in that the web-like connection takes the form of a common closure sleeve (1; 10), on which are provided two constricting tongues (3, 4) which, as the periphery varies, may be firmly clamped in the common closure sleeve (1; 10).

2. Double ring clip according to claim 1, characterized in that the constricting tongues (3, 4) may be fixed by means of locking parts (8, 9; 11, 12).

3. Double ring clip according to claim 2, characterized in that the locking parts have a saw toothing (8, 9).

4. Double ring clip according to claim 1, characterized in that the two constricting tongues (3, 4) are 1.2 mm wide and are at a constant distance apart of 1.2 mm.

5. Double ring clip according to claim 1, characterized in that micro-depressions (13) which are open at one end are provided on the surface of the clip.

6. Double ring clip according to claim 1, characterized in that the clip is made of a biodegradable material.

7. Double ring clip according to claim 1, characterized in that the clip is made of plastic.

8. Double ring clip according to claim 1, characterized in that the clip is made of metal.

9. Double ring clip according to claim 1, characterized in that the constricting tongues (3, 4) have an elastic coating.

## Revendications

1. Clip à deux anneaux pour l'obturation d'une trompe utérine, d'un canal déférent ou d'un vaisseau sanguin, comprenant deux languettes de serrage annulaires réunies en une unité par une liaison en forme de barrette et placées à distance constante l'une par rapport à l'autre, **caractérisé en ce** que la liaison en forme de barrette est conformée en douille de fermeture (1; 10) commune sur laquelle sont prévues deux languettes de pincement (3, 4) qui peuvent être bloquées dans la douille de fermeture (1; 10) commune avec variation de la circonférence.

2. Clip à deux anneaux selon la revendication 1, caractérisé en ce que les languettes de pincement (3, 4) peuvent être bloquées par des éléments d'arrêt (8, 9; 11, 12).

3. Clip à deux anneaux selon la revendication 2, caractérisé en ce que les éléments d'arrêt présentent un crantage en dents de scie (8, 9)

4. Clip à deux anneaux selon la revendication 1, caractérisé en ce que les deux languettes de pincement (3, 4) présentent une largeur de 1,2 mm et un écartement constant de 1,2 mm.

5. Clip à deux anneaux selon la revendication 1, caractérisé en ce que des microcavités (13) ouvertes sur un seul côté sont prévues sur la surface du clip.

6. Clip à deux anneaux selon la revendication 1, caractérisé en ce qu'il est réalisé dans unc matière biodégradable.

7. Clip à deux anneaux selon la revendication 1, caractérisé en ce qu'il est réalisé en matière plastique.

8. Clip à deux anneaux selon la revendication 1, caractérisé en ce qu'il est réalisé en métal.

9. Clip à deux anneaux selon la revendication 1, caractérisé en ce que les languettes de pincement (3,4) comportent un revêlement élastique.

Fig. 2   Fig. 1   Fig. 6   Fig. 7

Fig. 3   Fig. 4   Fig. 8   Fig. 10

Fig. 5   Fig. 9

EP 0 263 360 B1